# EUROPEAN PATENT APPLICATION

(11) **EP 4 261 538 A1**
(43) Date of publication of application: **18.10.2023**
(21) Application number: 22305568.2
(22) Date of filing: 15.04.2022
(51) Int. Cl.: G01N 33/50

(54) **METHODS FOR DETERMINING THE MOISTURE CONTENT OF THE SKIN**

(71) Applicant: Chanel Parfums Beauté, 92200 Neuilly-sur-Seine (FR)
(72) Inventor: BOUISSOU-CADIO, Emmanuelle, 93694 PANTIN CEDEX (FR)
(74) Representative: Plasseraud IP

(57) **Abstract**

The present invention relates to the identification of new biomarkers that are specifically associated with skin dryness. These biomarkers, which are extended synaptotagmin-3 (ESYT3), Ubiquinin-carboxyl terminal hydrolase 7 (USP7), periplakine (PPL) and Late Cornified Envelop Protein 1C (LCE1C), allow for the accurate evaluation of the moisture content of the skin, and particularly allow for the diagnosis of dry skin. The present invention pertains to a method for determining the moisture content of the skin, and particularly for diagnosing dry skin in a subject, said method comprising a step of measuring, in a skin sample obtained from said subject, the expression level of at least one protein selected from the group consisting of ESYT3, USP7, PPL and LCE1C. The present invention also relates to a method for evaluating the efficacy of a cosmetic treatment for hydrating the skin and to a method for screening candidate cosmetic compounds, both being based on measuring the expression level of the above biomarkers.

## Description

### Technical Field

The present invention pertains to methods for determining the moisture content of the skin based on the expression level of specific biomarkers.

### Background Art

The skin consists mainly of three layers, namely, starting from the most superficial, the epidermis, the dermis and the hypodermis. The outer layer of the skin, the epidermis, consists of keratinocytes (in the majority), melanocytes (involved in skin pigmentation) and Langerhans cells. Its function is to protect the body against the outside environment and to ensure its integrity, in particular to slow down the penetration of microorganisms or of chemical substances, and to prevent evaporation of the water contained in the skin.

To do this, keratinocytes undergo a continuous oriented maturation process during which the keratinocytes located in the basal layer of the epidermis form, at the terminal stage of their differentiation, corneocytes which are totally keratinized dead cells in the form of cornified envelopes consisting of proteins and lipids such as ceramides. This uppermost layer of the epidermis is referred to as the stratum corneum and is an essential component of the barrier function of the epidermis.

The barrier function of the epidermis can however be disrupted under certain climatic conditions (under the effect of cold and/or wind, for example), or else under the effect of stress, fatigue or ageing. Disruptions in the barrier function of the skin promote the penetration of allergens, of irritant agents or of microorganisms and directly lead to skin dryness, which is often accompanied little by feelings of discomfort such as tautness or redness.

In order to prevent this phenomenon or to correct it, it is known practice to apply to the skin cosmetic compositions containing hygroscopic agents, such as sugars or polyols, intended to capture the water present in the skin and thus slow down its evaporation.

Several hydrating compounds are known and available. However, these compounds act via different mechanisms of action and are therefore adapted to different skin types (such as dry, sensitive, combination skin...). Unfortunately, most people do not know their skin type and use inappropriate products.

Skin dryness is usually evaluated either clinically, by visual assessment, or by using a corneometer which aims at measuring the relative permittivity of upper skin layers. Unfortunately, both these methods face strong limitations. Visual evaluation of the skin depends upon the specialist performing the assessment and can therefore vary from one judge to another. Similarly, corneometer assessments are limited in terms of reproducibility: the results being closely linked to the pressure exerted and to the atmospheric conditions under which measurements are made.

There is therefore a need for new evaluation methods allowing for the correct determination of skin types. It would particularly be advantageous to identify specific markers associated with dry skin and that would also represent promising targets for preventing/reversing skin dryness processes.

### Summary

The invention is defined by the claims.

Using tape-stripped stratum corneum samples, the present inventor has identified very specific biomarkers that are associated with dry skin phenotypes. The inventor has further shown that these specific biomarkers can actually be directly targeted to prevent, limit or even reverse skin dryness processes. These biomarkers are therefore extremely promising tools for the assessment and treatment of skin dryness. These biomarkers can further be used for identifying interesting candidate cosmetic compounds for hydrating the skin and for evaluating their efficacy. The biomarkers identified in the context of the present invention are extended synaptotagmin-3 (ESYT3), Ubiquinin-carboxyl terminal hydrolase 7 (USP7), periplakine (PPL) and Late Cornified Envelop Protein 1C (LCE1C).

Accordingly, a first aspect of the present invention pertains to a method for determining the moisture content of the skin in a subject, said method comprising a step of measuring, in a skin sample obtained from said subject, the expression level of at least one protein selected from the group consisting of ESYT3, USP7, PPL and LCE1C. Such a method particularly allows detecting dry skins and therefore allows for the proper treatment of such a condition.

According to a second aspect, the present invention also relates to a method for evaluating the efficacy of a cosmetic treatment for hydrating the skin in a subject, said method comprising the steps of:
a) measuring, in a skin sample obtained from said subject before having received said cosmetic treatment, the expression level of at least one protein selected from the group consisting of ESYT3, USP7, PPL and LCE1C;
b) measuring, in a skin sample obtained from said subject after having received said cosmetic treatment, the expression level of said at least one protein;
c) comparing the expression levels measured in steps a) and b); and
d) determining that said cosmetic treatment is efficient when the expression level measured in step b) is lower than that measured in step a), or that said treatment is inefficient when the expression level measured in step b) is similar to or higher than that measured in step a).

Finally, the present invention further relates to a method for screening candidate cosmetic compounds for hydrating the skin, wherein said method comprises the following steps:
a) measuring, in a skin sample, the expression level of at least one protein selected from the group consisting of ESYT3, USP7, PPL and LCE1C;
b) bringing a test compound in contact with said skin sample;
c) measuring the expression level of said at least one protein in said skin sample; and
d) selecting the compound if the expression level measured in step c) is lower than that measured in step a)

### Description of Embodiments

The present invention relates to the identification of new biomarkers that are specifically associated with skin dryness. These biomarkers, which are extended synaptotagmin-3 (ESYT3), Ubiquinin-carboxyl terminal hydrolase 7 (USP7), periplakine (PPL) and Late Cornified Envelop Protein 1C (LCE1C), allow for the accurate evaluation of the moisture content of the skin, and particularly allow for the diagnosis of dry skin. The present inventor has particularly shown that these biomarkers are significantly overexpressed in dry skins as compared to normal skins, and that their expression level is directly associated with skin dryness. The inventor has further demonstrated that moisturizing treatments have a direct effect on the expression level of the biomarkers according to the invention, and, accordingly, that they represent an extremely promising tool for the treatment of dry skin and for the identification of new moisturizing agents.

The results disclosed herein show that:
- The expression level of ESYT3, USP7, PPL and LCE1C in a skin sample allows evaluating whether said skin is hydrated or not,
- These biomarkers are directly involved in dry skin processes and can therefore be suitable targets for preventing/treating dry skin, and
- The expression level of ESYT3, USP7, PPL and LCE1C can be used as a marker for identifying interesting cosmetic compounds for hydrating the skin.

Accordingly, a first aspect of the present invention pertains to a method for determining the moisture content of the skin, and particularly for diagnosing dry skin in a subject, said method comprising a step of measuring, in a skin sample obtained from said subject, the expression level of at least one protein selected from the group consisting of ESYT3, USP7, PPL and LCE1C. Such a method particularly allows detecting dry skins and therefore allows for the proper treatment of such a condition.

ESYT3, USP7, PPL and LCE1C are known proteins with which the skilled person is completely familiar.

**Extended synaptotagmin-3** (ESYT3) is a member of the extended synaptotagmins (Esyt) family which comprises multi-domain C2 membrane proteins whose orthologs are found in organisms ranging from yeast to humans. Three Esyt genes exist in mice and humans. They are known to be involved in the formation of junctions between the endoplasmic reticulum and the plasma membrane, as well as in the renewal of Ca²⁺-dependent membrane phospholipids. ESYT3 binds to glycerophospholipids in a barrel-like domain and is reported to possibly play a role in cellular lipid transport. The sequence of human ESYT3 is accessible under entry A0FGR9 in the Uniprot database.

**Ubiquinin-carboxyl terminal hydrolase 7** (USP7) has been shown to regulate the renewal of many signaling molecules, such as p53 and PTEN. Li et al (EMBO Rep. 2020 May 6; 21(5)) have shown that deletion of USP7 in keratinocytes leads to an increased ubiquitination of IKKa (IkB Kinase α) leading to a decrease in the level of IKKa during cell differentiation. Skin regenerated from transplanted USP7 KO cells present striking epidermal abnormalities, including thickened epidermis and basal cell expansion. Deletion of USP7 in epidermal keratinocytes leads to a decrease in IKKa level and to aberrant differentiation. The sequence of human USP7 is available under entry Q93009 in the Uniprot database.

**Periplakin** (PPL) is a component of the cornified envelope of keratinocytes. The corneal envelope is assembled from a series of precursor proteins that are crosslinked under the action of transglutaminases. The resulting structure is an insoluble layer of proteins with covalently attached lipids, having a thickness of -15-nm, and which is deposited at the inner surface of the plasma membrane in keratinocytes. Members of the plakin protein family, to which periplakin belong, serve as epidermal cytolinkers and components of cell-cell and cell-matrix adhesion complexes, i.e. desmosomes and hemidesmosomes, respectively. The sequence of human PPL is available under entry O60437 in the Uniprot database.

**Late Cornified Envelop Protein 1C** (LCE1C) is a precursor of the cornified envelope of the stratum corneum. LCE1C belongs to the late cornified envelope family whose members are integrated into the cornified envelope through transglutaminase-mediated cross-linking in the process of envelope maturation. The sequence of human LCE1C is available under entry Q5T751 in the Uniprot database.

In the context of the present invention, the expression level of at least one of these biomarkers is measured. The expression level of one of these biomarkers is sufficient for determining the moisture content of the skin. Nevertheless, the method according to the present invention can comprise measuring the expression level of 1, 2, 3 or of all these 4 biomarkers.

The person skilled in the art is familiar with many techniques that are used on a daily basis to determine the expression level of a protein.

The expression level of a protein can be evaluated by measuring the expression level of the gene encoding it. The expression level of a gene is typically determined by analyzing the mRNA(s) transcribed from that gene. These nucleic acid molecules can typically be extracted from the biological sample obtained from the subject and analyzed by standard methods. One commonly used method is to subject the isolated mRNAs to reverse transcription ("RT") coupled with polymerase chain reaction ("PCR") amplifications using oligonucleotide primers specific to the genes of interest. Quantification of mRNAs can typically be performed using one of two real-time quantification technologies called SYBR Green^{®} or TaqMan^{®}.

The expression level of the protein can also be directly evaluated by measuring the amount of said protein detected in the sample. Such methods typically involve contacting the biological sample to be analyzed with an agent capable of specifically binding the target protein. This agent is usually a polyclonal or monoclonal antibody. The presence of the protein is then typically detected by standard immunodetection methods after separation of the proteins by electrophoresis (technique also called "Western blotting") or by immunoassays by direct, indirect, competition or immunocapture methods (techniques also called "ELISA"). The formation of a complex between the protein of interest and the antibody(s) targeting said protein is usually detected and quantified by measuring an enzymatic reaction generating a colored, chemiluminescent or fluorescent product.

By **"moisture content of the skin",** it is herein referred to the amount of water comprised in the skin, and particularly in the stratum corneum. Our skin is made up of 70% water, which is distributed in a gradient decreasing from 70% in depth to reach 15% on its surface. This natural phenomenon, called skin perspiration, is possible thanks to a good-quality skin barrier which allows for the retention of water within the skin. A healthy skin barrier is based on a subtle balance between water (15%) and lipids (10-15%). Healthy and normal stratum corneum contains about 15-20 % water. Under these normal conditions, the skin is supple and soft. The moisture content of the skin allows classifying skins according to their water content. Skins are generally classified under four groups which are well-moisturized skin, normal/moisturized skin, dry skin and very dry skin. Dry skin is noted when the water content in the stratum corneum is lower than 15%, particularly lower than 10% and very dry skins when said content is of 5% and below. In a similar manner, normal/moisturized skins present a water content in the stratum corneum comprised between 15 and 20%, and said content is above 20% in well-moisturized skins. A dry skin is defined by a disequilibrium in the above balance, and particularly by a lack in lipids and a disorganization of its physical barrier causing a loss of water and a deterioration of cell maturation. This permanent skin condition is marked by tightness, redness and scales. Moisturizing compounds generally aim at compensating this lack of surface lipid with ceramides or with urea to reduce the transepidermal water loss.

The moisture content of the skin is a criterion that is routinely assessed by dermatologists and skin specialists. Several techniques are currently used for determining the moisture content of the skin. These techniques include e.g. the use of a corneometer, visual assessments or near infrared (NIR) spectroscopy (see e.g. for review Khazaka G., Bioengineering of the skin: water and stratum corneum (2005): 249; or Mohamad M. et al. Journal of Physics: Conference Series. Vol. 546. No. 1. IOP Publishing, 2014).

The method according to the present invention allows determining the moisture content of the skin. Therefore, in the context of the present invention, the method advantageously comprises a step wherein the skin is classified as being very dry, dry, normal/moisturized or well moisturized based on the expression levels of the present biomarkers.

As mentioned above, the present inventor has shown that the biomarkers according to the present invention are overexpressed in dry skins. The inventor has particularly shown that the more the biomarkers are expressed in a skin sample, the dryer the skin is. The method according to the present invention therefore particularly allows for the diagnosis, i.e. the detection, of dry skin (including very dry skin). Accordingly, according to a specific embodiment, the method according to the present invention comprises determining that the higher the expression level of said at least one protein is, the dryer the skin of the subject is.

Advantageously, the expression level of the biomarker according to the invention can be compared to a reference value. This reference value is determined for each biomarker. It can be determined experimentally, empirically or theoretically and is set in order to obtain optimal specificity and selectivity. This reference value can be determined on the basis of results obtained in a control population, for example in normal/moisturized skins or, alternatively, in dry skins. An expression level similar to a threshold value determined on the basis of normal/moisturized skins will be associated with a normal skin profile, whereas an expression level significantly different from this value, and in particular higher than this reference value, will be associated with a dry skin profile. Conversely, an expression level similar to a threshold value determined on the basis of dry skins will be associated with a dry skin profile, whereas an expression level significantly different, and in particularly lower than such a reference value, will be associated with a normal/moisturized skin profile.

According to a preferred embodiment, the reference value is determined on the basis of the expression level of the biomarkers according to the present invention measured in normal/moisturized skins. According to such an embodiment, the present invention pertains to a method for determining the moisture content of the skin in a subject, said method comprising the following steps:
a) measuring, in a skin sample obtained from said subject, the expression level of at least one protein selected from the group consisting of ESYT3, USP7, PPL and LCE1C;
b) comparing the expression level of said at least one protein with a reference value determined in normal/moisturized skins; and
c) concluding that the skin of the subject is dry or very dry when the expression level of said at least one protein is higher than the reference value.

The "**skin sample**" according to the present invention can be any skin sample in which the expression level of the proteins according to the present invention can be detected and measured. Typically, the skin sample according to the present invention is a stratum corneum sample. Stratum corneum samples can be obtained via various techniques including biopsies, skin scraping or tape striping (see for review Michaels, Barry. (2002). Chapter 26 - Skin Sampling Techniques: Handbook of Topical Antimicrobials and Their Applications). The sample according to the present invention is preferably obtained by using non-invasive techniques. According to a preferred embodiment, said stratum corneum sample is a tape-stripped stratum corneum sample. Typically, an adhesive tape is pressed onto the surface of the skin and is subsequently abruptly removed. This technique is routinely used in the field of dermatological research (see for review Hughes, A. J., et al. British Journal of Dermatology 185.1 (2021): 26-35).

The "**subject**" according to the present invention is a mammal, preferably a human.

According to a specific embodiment, the method according to the present invention further comprises measuring the expression level of interleukin-1 receptor antagonist protein (IL1RN). According to this specific embodiment, the method according to the invention comprises measuring the expression level of 1, 2 3 or 4 protein(s) selected from ESYT3, USP7, PPL and LCE1C and further comprises measuring the expression level of IL1RN. IL1RN is one of the inhibitory cytokines of the IL-1 family. It is expressed by keratinocytes and has anti-inflammatory effects. The sequence of human IL1RN is available under entry P18510 in the Uniprot database. The inventor has shown that the expression level of IL1RN is also correlated with dry/very dry skins.

As explained throughout the description, the expression level of the proteins according to the present invention is associated with the moisture content of the skin. These proteins are thus excellent markers for evaluating the impact of compounds on the moisture content of the skin, in particular for evaluating whether a compound allows improving the water content of the skin. The expression level of these proteins can thus be used for evaluating whether a moisturizing treatment is efficient, or, alternatively, for screening candidate cosmetic compounds.

Therefore, according to a further embodiment, the present invention also pertains to a method for evaluating the efficacy of a cosmetic treatment for hydrating the skin in a subject, said method comprising the steps of:
a) measuring, in a skin sample obtained from said subject before having received said cosmetic treatment, the expression level of at least one protein selected from the group consisting of extended synaptotagmin-3 (ESYT3), Ubiquinin-carboxyl terminal hydrolase 7 (USP7), periplakine (PPL) and Late Cornified Envelop Protein 1C (LCE1C);
b) measuring, in a skin sample obtained from said subject after having received said cosmetic treatment, the expression level of said at least one protein;
c) comparing the expression levels measured in steps a) and b); and
d) determining that said cosmetic treatment is efficient when the expression level measured in step b) is lowerthan that measured in step a), orthat said treatment is inefficient when the expression level measured in step b) is similar to or higher than that measured in step a).

The present invention also pertains to a method for screening candidate cosmetic compounds for hydrating the skin, wherein said method comprises the following steps:
a) measuring, in a skin sample, the expression level of at least one protein selected from the group consisting of extended synaptotagmin-3 (ESYT3), Ubiquinin-carboxyl terminal hydrolase 7 (USP7), periplakine (PPL) and Late Cornified Envelop Protein 1C (LCE1C);
b) bringing a test compound in contact with said skin sample;
c) measuring the expression level of said at least one protein in said skin sample; and
d) selecting the compound if the expression level measured in step c) is lower than that measured in step a).

The candidate cosmetic compound may be of any type. It may be of natural origin or may have been produced by chemical synthesis. This may involve a library of structurally defined chemical compounds, uncharacterized compounds or substances, or a mixture of compounds.

Natural compounds include compounds from vegetal origin, like plants. Preferably, the candidate cosmetic compound is vegetal, preferably chosen from botanical extracts.

According to a preferred embodiment, the skin sample used in the context of the screening method according to the present invention is an artificial skin sample. Artificial skin is largely used in the field of cosmetic research (see e.g. Brohem et al. Pigment cell & melanoma research 24.1 (2011): 35-50; or Yun et al. Journal of Pharmaceutical Investigation 48.2 (2018): 215-223) and can be easily obtained from specialized manufacturers.

The present disclosure also provides a method for the treatment of dry skin, said method comprising a step of treating a skin identified as being dry according to the methods of the present invention with an effective amount of a hydrating compound.

The invention will be further illustrated by means of the following non-limiting examples.

### Examples

### Example 1: Identification of dry skin biomarkers:

The study was conducted on 37 women aged 35 to 40 years with normal skin (17) or very dry skin (20) on the leg. The skin condition of the women was classified by different parameters; visually under dermatological control by using grades scale of dry skin, by measurement of TWEL, capacitance and by sequential tape strip sampling. Staining of tape strips allows categorizing different grades of dry skin according to the distribution of corneocytes on the tape strip, their number and color intensity. The lysis of twelve tape strips was performed for proteomic identification and quantification. This proteomic characterization allowed identifying four new biomarkers that are preferentially expressed at the surface of the dry skin.

### 1- Material and Methods

Cohort Description: The purpose of the study was to reveal targets involved exclusively in dry skin. In order to be able to identify these targets a cohort of women with normal skin versus very dry skin was selected. This study brings together 37 volunteers aged 35-40 years with a phototype ranging from 1 to 3, divided into two subpopulations: one of 17 women with normal skin, and the other of 20 women with visibly very dry skin. This distribution was made by a dermatologist who evaluated the sampling area, front of the leg, in relation to its visual appearance on the day of the study. Confirmation on photographic support correlated with biophysical measurements such as TWEL and capacitance.

Description of the sampling method: The tape stripping, a non-invasive method, makes it possible to remove the first layers of the stratum corneum. The first tape strip is placed on the front of the leg and a standardized weight is then applied as a constant and repeatable pressure. The weight is applied for 10 seconds and then lifted. The contour of the tape strip is drawn so as to apply the following tape strip at the same location. The tape strip is then removed by using a clip in one go with a frank gesture. The first tape strip is discarded in order to standardize for the following the quantity taken. The next tape strips are then applied and removed in a similar manned as the first one. The tape strips are then stored at -80°C. A total of 15 tape strips on 3 distinct areas were sampled. Twelve of them were used for protein extraction and one for morphological staining.

Tape strip staining: Tape strips were colored so as to highlight the distribution and morphology of corneocytes for each volunteer. Tape strips were thawed at room temperature, placed on a hot plate at 37°C and then covered with Stain PMS: Mixture of Basic Fuchsin and Toluidin Blue during 1 hour. The stained tape strips are then rinsed in successive baths of tap water and left to dry in the open air and then placed between slides and lamella for further analysis.

Image analysis: Slide scans were performed by using the ScanScope^{®} AT Turbo slide scanner, at x40 magnification for a resolution of 0.25 µm/pxl. Based on color deconvolution, two algorithms have been developed to:
1) Measure the tissue surface positive to staining and classify the surface of positive corneocytes according to 3 classes of colorimetric intensities. The algorithm applies a colorimetric intensity gradient in its positive tissue surface measurement. It is therefore sufficient to classify the surface of corneocytes into three groups: corneocytes weakly, moderately and strongly marked. Finally, the algorithm determines for each tape strip a normalized value of average optical density corresponding to the average intensity of corneocytes. The threshold values and parameters used for the measurement of the surface of corneocytes and the classification according to a color gradient are the same for each tape strip.
2) Count the islets formed by corneocytes and measure their areas. First, the analysis area and possible exclusion zones are determined. The color deconvolution is then applied to extract the positive tissue from the rest of the image and thus free itself from artifacts that can alter the analysis of images. Finally, the algorithm will segment and individualize the islets so that they can be counted, and their areas measured. The ratio between the total area analyzed and the area occupied by the islets allows quantifying the density of the islets per tape strip as a percentage value.

Protein extraction: Each tape was placed, adhesive side up, in its own 20-mL borosilicate scintillation vial (Wheaton; VWR). Tapes were soaked in PBS buffer containing SDS (0.2%), Propylene glycol (0.5%) and HALT anti-protease cocktail (1X). Vials were placed in a sonication bath for60 minutes. Cell lysates were collected from each vial and pooled according to donor's number. Proteins were precipitated overnight at -20°C in a solution of 90% Methanol. Precipitated proteins were collected by centrifugation (15000g, 20 minutes, 4°C) and dissolved in Tris-HCI buffer containing urea (8M). Protein concentrations were determined using BCA method.

Protein purification and concentration: Peptide extracts were prepared according to FASP method (Filter Aided Sample Preparation). 50µg of proteins were loaded onto an ultrafiltration device (Amicon Ultra 0.5, 10kDa MWCO), then reduced (Dithiothreitol), alkylated (iodoacetamide) and digested by trypsin. Peptides were purified by SPE chromatography (C18), dried and solubilized in 100µl of 0.1% formic acid aqueous solution. Peptide concentration was determined using BCA method.

Protein Analysis LC-MS/MS « shotgun proteomics »: 250ng peptides were injected in triplicate for each sample when possible. For samples with undetermined peptide concentrations, 10 µl of undiluted sample were used. Chromatography was performed using Ultimate 3000 (Dionex) equipment using C18 (75pm x 50cm, 2µm material) column applying a 2.5% to 35% acetonitrile 60 minutes gradient at a flow rate of 300nl/min after a 3-minute trapping step on precolumn. Data were acquired using the Q-Exactive Plus (Thermo) mass spectrometer. MS scan was performed with a resolution of 70000 and an accumulation time of 60 ms. MS/MS scan was performed with a resolution of 17500 on the 10 most intense ions of each cycle with an accumulation time of 60ms. 5600 cycles were performed, thus an average of 14 cycles per chromatographic peak. The following experimental settings were applied: Source voltage (2300V) Sweep gas (0 psi) Transfert tube temperature (275°C) Normalized collision energy 28* (*Collision energy is automatically determined depending on the mass and the charge of analyzed compound).

Protein identification: Proteins were identified using SEQUEST-HT algorithm and a database gathering Human reference proteome mined from UNIPROT. Search parameters were: enzyme = trypsin (full); allowed iscleavage =2; precursor error tolerance = 10 ppm; fragment error tolerance = 0.02 Da ; Dynamic modification = oxidation (M), deamidation (N/Q) ; Protein terminus modification = acetylation ; Static modification = carbamidomethyl (C). False Discovery Rate (FDR) determination was made using Percolator algorithm.

Protein quantification: Data were processed using Minora and feature mapper for Proteome Discoverer 2.2 software. Peak integration parameters were: Post acquisition recalibration=True (fine parameters) Minimum trace length =5; Minimum number of isotope=2; max Delta RT for isotope=0.2 min PSM confidence level for integration = High. Chromatographic alignment parameters were: RT alignment= TRUE; Parameter tuning=Fine; max RT shift= 5 min mass tolerance 10 ppm. Feature mapping parameters were: RT tolerance=automatic; mass tolerance=automatic; S/N threshold=2. Statistical analyses were performed by using Precursors Ions quantifier node for Proteome Discoverer 2.2 software. General Quantification Settings were: Peptide to use= Unique + RAZOR (Unique = peptides that are not shared by different proteins or protein groups ; RAZOR = peptides shared by multiple protein's groups but only used to quantify protein with the largest number of unique peptides and with the longest amino acid sequence); Consider Proteins Groups for Peptide Uniqueness=True; Reject Quan Results with Missing Channels=False. Precursor Quantification Settings were: Precursor Abundance Based on Area; Min number Replicate feature = 50% (peptides must be detected in at least 50% of sample of one group for be use in quantification). Normalization settings: Total Peptide amount (Calculates the total sum of abundance values for each injection over all peptides identified, the injection with the highest total abundance is used as reference to correct abundance values in all other injections by a constant factor per injection, so that at the end the total abundance is the same for all injections). Quan Rollup Hypothesis Testing settings: Ratio Calculation=Summed abundance based (Protein Ratios are calculated from median of summed sample abundances of replicate group). Imputation Mode=Replicate Based Resampling (Missing values are replaced with random values sampled from distributions centered around medians of detected values of (technical, biological) replicates). Hypothesis Test= ANOVA (individual proteins).

### 2/ Results:

### Tape strips staining: Morphological analysis.

Tape strips staining provided extensive information ranging from the surface occupied by the corneocytes on the entire tape strip to the number of islets formed by them and the associated colorimetric intensity. Indeed, dry skins were gradually associated with larger surfaces occupied by the corneocytes on the tape strip. Dry skins were also gradually associated with increased islets numbers of high colorimetric concentration. The colorimetric density and dense distribution of corneocytes is directly correlated to the degree of dryness of the skin.

### Quantitative analysis of the stained tape strips

Further to the analysis made by the dermatologist and the correlation of said analyses with biophysical measurements, a population of normal skin and a population of people with dry skin were determined. After applying the same distribution, these results were correlated with those obtained regarding the average size of the islets obtained on the entire tape strip.

Dry skin corneocytes occupy more than 43% of the tape strip surface as compared to normal skin. The surface of the islets is on average increased by 28% in dry skin. Dry skin presents a high colorimetric intensity that is 255% higher than that observed in normal skin.

### Proteomic characterization in dry skin:

Analysis of dry and normal skin reveals that out of 613 proteins identified, 340 are differentially regulated in dry skin. An average of 227 and 212 proteins were identified respectively in sample from Dry Skin group and Normal Skin Group. From a quantitative point of view, among the 340 quantifiable proteins (after curation), 88 were considered as over-expressed in sensitive skin while 77 were considered as under-expressed (thresholds: p-value <0.05, fold changes >1.5 or <0.67).

### Specific protein biomarkers of dry skin:

By applying an imputation percentage of 100% to normal skin, we obtain by contrast a very low percentage in dry skin. This low percentage of imputation is inversely proportional to the expression of the target in dry skin. As a result, this means that the lower the percentage of imputation, the more the target is expressed in the skin. This exercise allowed selecting targets that are preferentially expressed in dry skin. The 5 main targets present exclusively in dry skin are: periplakin, ESYT3, LCE1C, USP7 and IL1RN.

The overexpression of these different targets at the keratinocyte level allows for the creation of a new model for the study of dry skin. The action of the selected active ingredients on these targets can then be evaluated to evaluate their moisturizing effects.

### Example 2: Effects of a moisturizing agent on the selected biomarkers:

The overexpression of the four proteins mentioned characterizes dry skin. As a result, an active ingredient reducing their overexpression would allow dry skin to return to a normal state. This postulate was tested for LCE1C. The impact of a systemic treatment of the active Anthyllis at two different concentrations 0.1% and 0.033% on melanized reconstructed epidermis was determined. After 5 days of treatment, the expression of LCE1C is decreased by 4.1 and 11.8 times, respectively, as compared to the untreated control.

The biomarkers according to the present invention therefore allow accurately diagnosing dry skin and represent extremely relevant targets for evaluating the efficacy of hydrating compounds or for monitoring the efficacy of a hydrating treatment.

## Claims

1. A method for determining the moisture content of the skin in a subject, said method comprising a step of measuring, in a skin sample obtained from said subject, the expression level of at least one protein selected from the group consisting of extended synaptotagmin-3 (ESYT3), Ubiquinin-carboxyl terminal hydrolase 7 (USP7), periplakine (PPL) and Late Cornified Envelop Protein 1C (LCE1C).

2. The method according to claim 1, said method further comprising a step wherein said skin is classified as a very dry, dry, moisturized or well-moisturized based on the expression level measured.

3. The method according to claim 1 or 2, wherein it is determined that the higher the expression level of said at least one protein is, the dryer the skin of the subject is.

4. The method according to any one of claims 1 to 3, wherein said method further comprises a step of measuring, in said skin sample obtained from the subject, the level of interleukin-1 receptor antagonist protein (IL1RN).

5. The method according to any one of claims 1 to 4, wherein said skin sample is a stratum corneum sample.

6. The method according to claim 5, wherein said stratum corneum sample is a tape stripped stratum corneum sample.

7. A method for evaluating the efficacy of a cosmetic treatment for hydrating the skin in a subject, said method comprising the steps of:
a) measuring, in a skin sample obtained from said subject before having received said cosmetic treatment, the expression level of at least one protein selected from the group consisting of extended synaptotagmin-3 (ESYT3), Ubiquinin-carboxyl terminal hydrolase 7 (USP7), periplakine (PPL) and Late Cornified Envelop Protein 1C (LCE1C);
b) measuring, in a skin sample obtained from said subject after having received said cosmetic treatment, the expression level of said at least one protein;
c) comparing the expression levels measured in steps a) and b); and
d) determining that said cosmetic treatment is efficient when the expression level measured in step b) is lower than that measured in step a), or that said treatment is inefficient when the expression level measured in step b) is similar to or higher than that measured in step a).

8. A method for screening candidate cosmetic compounds for hydrating the skin, wherein said method comprises the following steps:
a) measuring, in a skin sample, the expression level of at least one protein selected from the group consisting of extended synaptotagmin-3 (ESYT3), Ubiquinin-carboxyl terminal hydrolase 7 (USP7), periplakine (PPL) and Late Cornified Envelop Protein 1C (LCE1C);
b) bringing a test compound in contact with said skin sample;
c) measuring the expression level of said at least one protein in said skin sample; and
d) selecting the compound if the expression level measured in step c) is lower than that measured in step a).

9. The method according to claim 8, wherein said candidate cosmetic compound is a botanical extract.

10. The method according to claim 8 or 9, wherein said skin sample is an artificial skin sample.
